# EUROPEAN PATENT APPLICATION

(11) **EP 4 552 615 A1**
(43) Date of publication of application: **14.05.2025**
(21) Application number: 23208756.9
(22) Date of filing: 09.11.2023
(51) Int. Cl.: A61F 2/46, A61F 2/38, A61F 2/30

(54) **TIBIAL TRIAL INSERT SYSTEM**

(71) Applicant: Aesculap AG, 78532 Tuttlingen (DE)
(72) Inventor: Zouaghi, Housseyn, 52000 Chaumont (FR); Moussa, Said, 52000 Chamarandes-Choignes (FR)
(74) Representative: Patentanwälte Ruff, Wilhelm, Beier, Dauster & Partner mbB

(57) **Abstract**

A tibial trial insert system (1) comprising:

a bearing component (100) having a superior articulating surface (101) and an inferior surface (102);

a plate component (200) having a superior surface (201) and an inferior surface (202);

a spacing adjustment assembly (300) configured to be arrangeable between the bearing component (100) and the plate component (200), the spacing adjustment assembly (300) having at least one superior connector element (301) configured to removably engage with the bearing component (100), at least one inferior base element (302) configured to removably engage with the plate component (200), and having a coupling arrangement (C) movably coupling the connector element (301) and the base element (302) in proximal/distal direction and to a limited extent; and

a plurality of shims (500, 600, 700), each shim (500, 600, 700) configured to be slidable between the connector element (301) and the base element (302) to adjust a proximal/distal height of the spacing adjustment assembly (300), in order to thereby adjust a relative proximal/distal spacing between the bearing component (100) and the plate component (200).

## Description

### TECHNICAL FIELD AND PRIOR ART

The present invention relates to a tibial trial insert system.

During a total knee arthroplasty, tibial trial insert systems are typically used to assist a surgeon in determining a size, shape or other configuration of a permanent prosthesis for replacing a portion of the natural knee joint. In particular, such tibial trial insert systems are used to determine a relative spacing between a femoral component and a tibial component of the permanent prosthesis.

US 2015/0359642 A1 discloses a tibial trial insert system comprising a bearing component having a superior articulating surface and an inferior surface; a base component having a superior surface and an inferior surface, the base component configured to removably engage with the bearing component; and a plurality of shims, each shim configured to be slidable between the inferior surface of the bearing component and the superior surface of the base component to change a relative proximal/distal spacing between the bearing component and the base component.

### SUMMARY OF THE INVENTION

It is an object of the present invention to provide an alternative tibial trial insert system.

According to the invention, the tibial trial insert system comprises: a bearing component having a superior articulating surface and an inferior surface; a plate component having a superior surface and an inferior fixation surface; a spacing adjustment assembly configured to be arrangeable between the bearing component and the plate component, the spacing adjustment assembly having at least one superior connector element configured to removably engage with the bearing component, at least one inferior base element configured to removably engage with the plate component, and having a coupling arrangement movably coupling the connector element with the base element in proximal/distal direction and to a limited extent; a plurality of shims, each shim configured to be slidable between the connector element and the base element to adjust a proximal/distal height of the spacing adjustment assembly, in order to thereby adjust a relative proximal/distal spacing between the bearing component and the plate component. The invention avoids, in particular, incorrect adjustment of the proximal/distal spacing between the bearing component and the plate component. To this end, the spacing adjustment assembly only allows a limited proximal/distal relative movement between the bearing component and the plate component. For achieving said limited movement, the connector element and the base element are movably and captively coupled by means of the coupling arrangement. Said limited movement prevents an insertion of an excessive number of shims and thus the setting of too large a proximal/distal height. As a result, the invention helps to prevent errors during surgery and improves patient safety. Moreover, due to the movable yet captive coupling between the connector element and the base element, the spacing adjustment assembly can be handled easily during surgery, in particular in comparison to a mere arrangement of loose and/or non-pre-assembled parts. Preferably, the spacing adjustment assembly is configured to be arrangeable between the inferior surface of the bearing component and the superior surface of the plate component. Preferably, each shim is configured to be slidable between an inferior surface of the connector element and a superior surface of the base element. Preferably, the connector element and the base element are fixed relative to each other by means of the coupling arrangement in anterior/posterior and/or medial/lateral direction. Engagement of the connector element with the bearing component and engagement of the base element with the plate component restrains an anterior/posterior movement and/or a medial/lateral movement between the bearing component and the plate component. For engagement with the base element, the plate component preferably comprises a recess configured for accommodating at least a portion of the base element and for restraining the base element in anterior/posterior direction and/or medial/lateral direction relative to the plate component. For engagement with the connector element, the bearing component preferably comprises an engagement portion which interacts with a complementary engagement portion of the connector element, thereby forming a plug-in, latching and/or snap-in connection. Preferably, the movability between the connector element and the base element in proximal/distal direction is limited by means of an end stop or the like. In this document, the terms "superior", "inferior", "anterior", "posterior", "medial", "lateral", "proximal" and "distal" are used according to their standard anatomical definitions. In this document, the phrase "proximal/distal spacing" denotes a spacing extending in a proximal and/or distal direction. Analogously, the phrase "anterior/posterior" means anterior and/or posterior; the phrase "medial/lateral" means medial and/or lateral. The plate component can also be termed "tibial plateau component".

In one embodiment, the coupling arrangement forms a telescopic mechanism which is extendable in proximal direction and retractable in distal direction. Providing the coupling arrangement in the form of a telescopic mechanism allows for a simple and robust design. The telescopic mechanism is telescoping in proximal/distal direction and, in this respect, extendable in proximal direction and retractable in distal direction. The telescopic mechanism is fixed to the base element on one end and fixed to the connector element on the other end. The telescopic mechanism is extendable in proximal direction by means of sliding at least one shim of the plurality of shims between the connector element and the base element. Starting from an extended position, the telescopic mechanism is retractable in distal direction by means of removing at least one shim of the plurality of shims between the connector element and the base element. Preferably, the telescopic mechanism comprises at least one cylinder element which is fixedly connected to the connector element and slidably received in a receiving bore of the base element. Hence, the telescopic mechanism has a single stage. Other embodiments can comprise two, three, four, five, six or even more cylinder elements, forming a telescopic cylinder with an according number of stages. Preferably, the proximal/distal movability of the cylinder element within the receiving bore of the base element is limited by means of a proximal end stop and/or a distal end stop. Preferably, the cylinder element and the receiving bore each have a non-circular, preferably oval or rectangular, cross-section, thereby preventing a relative rotation between the connector element and the base element about a proximal/distal axis. The non-circular cross-sections restrain a relative rotation between the connector element and the base element and, thus, between the bearing component and the plate component. Preferably, the telescopic mechanism is extendable between 0 mm and 16 mm, inclusive.

In one embodiment, the telescopic mechanism comprises a latching device configured for releasably latching the cylinder element in a fully retracted position relative to the base element. Releasably latching the cylinder element to the base element by means of the latching device in said fully retracted position leads to an improved stability and a more compact design when inserting the tibial trial insert system into the patient's knee. The latching can be released by extending the telescopic mechanism in proximal direction, i.e. by pushing or pulling the bearing component in proximal direction, e.g. by sliding at least one shim of the plurality of shims between the connector element and the base element.

In one embodiment, the latching device comprises at least one resilient matching member connected to the cylinder element and at least one latching portion formed on the base element and configured for latching interaction with the resilient latching member to releasably latch the cylinder element to the base element. Providing the latching device with at least one resilient latching member and at least one latching portion allows for a simple and robust design. The at least one resilient latching member and the at least one latching portion form said releasable latching connection in the fully retracted position of the cylinder element/telescopic mechanism.

In one embodiment, the cylinder element comprises at least one receiving bore, wherein the resilient latching member comprises at least one head portion extending between a first end and a second end, and at least one compression spring member, the head portion and the compression spring member both being received in said receiving bore of the cylinder element, wherein the first end of the head portion is configured to latch with the latching portion of the base element, and wherein the compression spring member acts on the second end of the head portion to bias the head portion into a latching position in which the first end of the head portion protrudes from the receiving bore of the cylinder element to latch with the latching portion of the base element. The inventors have found that providing the resilient latching member with at least one head portion and at least one compression spring member leads to a simple and robust design that is well suited for invasive applications. Preferably, the at least one receiving bore extends in medial/lateral direction. Alternatively, the at least one receiving bore extends in anterior/posterior direction. The head portion extends coaxially with the receiving bore and is biased by means of the at least one compression spring member. Said bias acts into the latching position. In one embodiment, a first end of the at least one compression spring member acts on the second end of the head portion and a second end of the compression spring member is supported on a portion of the cylinder element. In an alternative embodiment, the resilient latching member comprises two head portions, wherein the compression spring member is arranged axially between said two head portions for acting on their respective second end.

In one embodiment, the resilient latching member comprises a first head portion and a second head portion opposite in medial/lateral direction, wherein the base element comprises a first latching portion and a second latching portion opposite in medial/lateral direction, wherein the compression spring member is located between said first head portion and second head portion and acts on their respective second end, thereby biasing the first head portion into a latching position in which a first end of the first head portion protrudes from the receiving bore to latch with the first latching portion, and thereby biasing the second head portion into a latching position in which a first end of the second head portion protrudes from the receiving bore to latch with the second latching portion. Both the first head portion and the second head portion are received in the receiving bore of the cylinder element. The compressing spring member is located between the first head portion and the second head portion and biases the first head portion and the second portion into opposite directions. Providing the resilient latching member with two opposite head portions and the base element with two opposite latching portions allows for an improved latching connection. Said improved latching connection has two connection "points", i.e. firstly between the first head portion and the first latching portion and secondly between the second head portion and the second latching portion.

In one embodiment, the tibial trial insert system further comprises a handling instrument having an elongate shaft and a coupling mechanism arranged distally on said elongate shaft, wherein the coupling mechanism is configured for releasable coupling to an anterior coupling portion of the spacing adjustment assembly. The handling instrument allows for a simplified and ergonomic handling of the spacing adjustment assembly. The handling instrument can be used to arrange the spacing adjustment assembly between the bearing component and the plate component. The elongate shaft extends between a distal end and a proximal end. The coupling mechanism is arranged on the distal end of the elongate shaft. The coupling mechanism is configured for manual operation. For this purpose, an operating element is preferably provided and configured for manually shifting the coupling mechanism between a coupling state and a release state. Said operating element is preferably a button, a switch, a slider or the like. In a preferred embodiment, the anterior coupling portion of the spacing adjustment assembly is formed on the connector element. In an alternative embodiment, the anterior coupling portion is formed on the base element. In yet another embodiment, the anterior coupling portion is formed on the coupling arrangement.

In one embodiment, the anterior coupling portion is formed on the connector element of the spacing adjustment assembly, wherein the connector element is made of a metal material. Providing the anterior coupling portion on the connector element and making the latter of a metal material allows for an improved strength of the coupling between the coupling mechanism and the coupling portion. It is also possible to provide the anterior coupling portion on the bearing component. However, the bearing component is preferably made of a plastic material and a possible coupling between the coupling mechanism and a coupling portion of the bearing component is therefore less stable and robust in comparison to a coupling between the coupling mechanism and the coupling portion of the metal made connector element. Preferably, the anterior coupling portion forms a protrusion protruding in anterior direction.

In one embodiment, the coupling mechanism is configured for releasable coupling to an anterior coupling portion of each of the plurality of shims. Configuring the coupling mechanism for releasable coupling to each of the plurality of shims allows for a simplified and ergonomic handling of the shims as well. Hence, the handling instrument can be used for arranging the spacing adjustment assembly between the bearing component and the plate component and for sliding the shims between the connector element and the base element.

In one embodiment, the tibial trial insert system further comprises a securing component configured to be supported on and/or in a support portion of the bearing component and configured for detachable attachment to an attachment portion of the connector element of the spacing adjustment assembly, thereby releasably securing the removable engagement between the bearing component and the connector element. Providing the tibial trial insert system with said securing component allows for an improved connection between the bearing component and the spacing adjustment assembly. For this purpose, the securing component can be supported on and/or in said support portion of the bearing component and can be attached to said attachment portion of the connector element. The detachable attachment between the securing component and the attachment portion can be, e.g., in the form of a plug-in, latching and/or snap-in connection. The securing component secures the engagement between the bearing component and the connector element at least in proximal/distal direction.

In one embodiment, the attachment portion and/or the connector element is made of metal material. Making the attachment portion and/or the connector element of a metal material provides for an improved strength and durability of the detachable attachment of the securing component to the attachment portion/connector element. The attachment portion of the connector element can have any form, shape or size suitable for the present purpose.

In one embodiment, the support portion of the bearing component comprises a recessed support surface which is sunk distally into the articulating surface and a support opening extending between the support surface and an inferior surface of the bearing component, wherein the attachment portion of the connector element comprises an attachment opening positioned distally underneath and in line with the support opening, and wherein the securing component comprises a plug body configured to be plugged on the recessed support surface and a hook member configured to reach through the support opening and to positively engage with the attachment opening. The recessed support surface is preferably flat and configured to support the plug body of the securing component. The support opening is extending between the support surface and the inferior surface of the bearing component and, hence, a throughopening. The attachment opening is positioned underneath and in line with the support opening and configured to positively engage with the hook member. When the securing component is attached, the hook member reaches through the support opening and positively engages with the attachment opening, thereby securing the engagement between the bearing component and the connector element. The plug body defines a shape and size of the securing component. The hook member serves as attachment means for attaching the securing component to the connector element. The plug body and the hook member are fixedly connected. In one embodiment, the plug body and the hook member form a unitary one-piece component. In another embodiment, the plug body and the hook member are made from different materials and form separate parts of the securing component.

In one embodiment, the hook member is made of a metal material and/or the plug body is made of a plastic material. Making the hook member of a metal material provides for an improved strength of the connection between the hook member and the attachment portion. Preferably, the plastic material of the plug body is identical to a plastic material of the bearing component. This allows for identical material properties, e.g. frictional properties.

In one embodiment, the securing component comprises a locking device configured for releasably locking to a locking portion of the bearing component. Providing the securing component with said locking device allows for an improved connection between the securing component and the bearing component.

In one embodiment, the locking portion of the bearing component comprises a locking slit extending in a transversal plane and the locking device of the securing component comprises a flat locking member configured to be rotatable in said transversal plane relative to the securing component between a locking position in which the flat locking member positively engages the locking slit and a release position in which the flat locking member does not positively engage the locking slit. The locking slit of the bearing component extends in said transversal plane and, hence, in medial/lateral and anterior/posterior direction. The flat locking member is rotatably mounted on the securing component and, in particular, rotatable relative to the plug body and/or hook member. The flat locking member is rotatable between said locking position and said release position. Rotating the flat locking member into the release position releases the locking between the securing component and the bearing component to detach the securing component from the attachment portion of the connector element.

### BRIEF DESCRIPTION OF THE DRAWINGS

In the following, preferred exemplary embodiments of the invention will be described in detail with reference to the drawings. Throughout the drawings, the same elements will be denoted with the same reference numerals. The drawings schematically show:
- fig. 1: in a perspective view, an embodiment of a tibial trial insert system having a bearing component, a plate component, a spacing adjustment assembly and a plurality of shims;
- fig. 2: a perspective view of the spacing adjustment assembly of the tibial trial insert system according to fig. 1;
- fig. 3: the spacing adjustment assembly of fig. 2 in a perspective exploded view;
- fig. 4: the spacing adjustment assembly of figs. 2 and 3 in longitudinal sectional view with medial/lateral viewing direction;
- fig. 5: a perspective view of a handling instrument configured to handle the components of the tibial trial insert system according to fig. 1;
- fig. 6: a perspective view of a first handling situation in which the handling instrument of fig. 5 is used to handle the spacing adjustment assembly for engagement with the bearing component;
- fig. 7: in a perspective view, a further handling situation in which the handling instrument is used to handle the bearing component together with the spacing adjustment assembly for engagement with the base component;
- fig. 8: in a perspective view, a further handling situation in which the handling instrument is used to slide one of the plurality of shims between the bearing component and the plate component to increase a proximal/distal spacing between said components;
- fig. 9, 10, 11: in top views, different shims of the plurality of shims;
- fig. 12: in a further perspective view, the tibial trial insert system of fig. 1 together with a securing component, wherein the securing component is detached from the bearing component and the spacing adjustment assembly;
- fig. 13: in a bottom view, the bearing component together with the attached securing component;
- fig. 14: in a detailed perspective view, the securing component, wherein a locking device of the securing component occupies a release position;
- fig. 15: in a further perspective view, the securing component, wherein the locking device occupies a locking position;
- fig. 16: the securing component of figs. 14 and 15 in a bottom view with the locking device occupying the locking position;
- fig. 17: a bottom view of the tibial trial insert system with attached securing component and without the plate component;
- fig. 18: the tibial trial insert system with attached securing component in a longitudinal sectional view and without the plate component, and
- fig. 19: the tibial trial insert system with a variant of the securing component attached in a further longitudinal sectional view and without the plate component.

### DETAILED DESCRIPTION OF EMBODIMENTS OF THE INVENTION

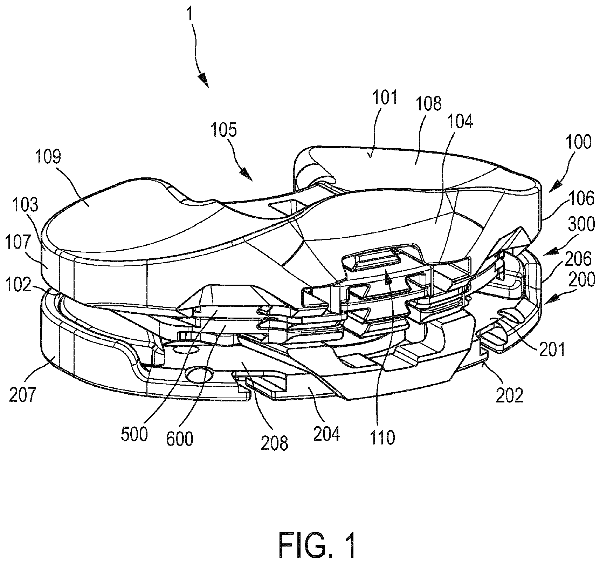

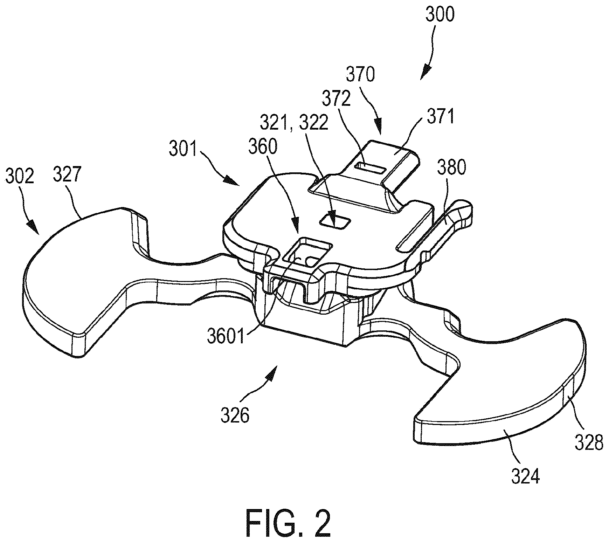

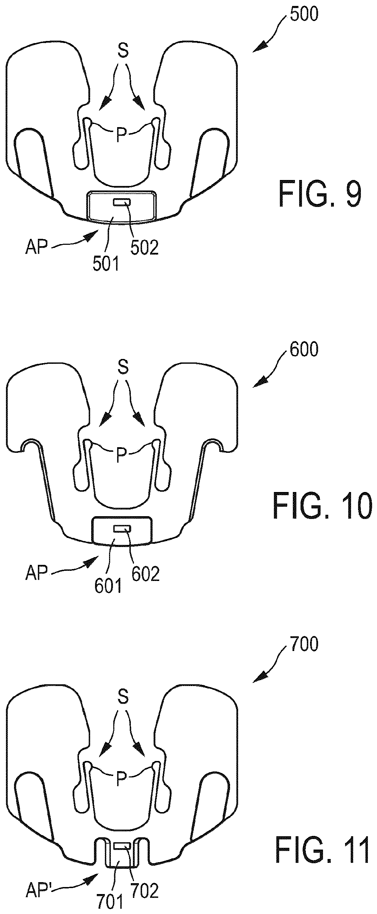

According to fig. 1, a tibial trial insert system is provided for use in a knee joint replacement surgery. The tibial trial insert system 1 comprises a bearing component 100, a plate component 200, a spacing adjustment assembly 300 (shown in detail in figs. 2, 3, 4) and a plurality of shims 500, 600, 700 (shown in detail in figs. 9, 10, 11), wherein fig. 1 only shows shims 500, 600 of said plurality of shims 500, 600, 700.

The bearing component 100 has a superior articulating surface 101, an opposing inferior surface 102 and a peripheral wall 103 extending from the inferior surface 102 to the superior articulating surface 101. The bearing component 100 further includes an anterior side 104, a posterior side 105, a lateral side 106 and a medial side 107. The superior articulating surface 101 is configured to articulate with natural or prosthetic condyles of a distal femur and includes a lateral articulating surface portion 108 and a medial articulating surface portion 109.

The plate component 200 has a superior surface 201, an opposing inferior fixation surface 202 and a peripheral wall 203 extending from the inferior fixation surface 202 to the superior surface 201. The plate component 200 further includes an anterior side 204, a posterior side (without reference sign), a lateral side 206 and a medial side 207. The inferior fixation surface 202 is configured for direct or indirect fixation to a proximal end of a tibia. The plate component 200 can also be referred to as tibial plateau component.

The spacing adjustment assembly 300 is configured to be arrangeable between the bearing component 100 and the plate component 200, in particular between the inferior surface 102 of the bearing component 100 and the superior surface 201 of the plate component 200. The spacing adjustment assembly 300 comprises a superior connector element 301, an inferior base element 302 and a coupling arrangement C.

The superior connector element 301 is configured to removably engage with the bearing component 100. The inferior base element 302 is configured to removably engage with the plate component 200. The coupling arrangement movably couples the connector element 301 with the base element 302 in proximal/distal direction and to a limited extent. In other words, the connector element 301 and the base element 302 are movably and captively coupled to each other by means of the coupling arrangement C.

The spacing adjustment assembly 300 is intended for adjusting a relative proximal/distal spacing between the bearing component 100 and the plate component 200. In other words, the spacing adjustment assembly 300 is intended to position the superior articulating surface 101 and/or the bearing component 100 in different height levels relative to the plate component 200, in particular relative to the inferior fixation surface 202. Such a spacing or height adjustment is required for trial reposition in knee joint replacement surgery. Said trial reposition is an operation step preceding the actual knee joint replacement, wherein sizes, dimensions and/or shapes of the tibial and femoral implant components required for the replacement of the knee joint are determined. This application related background of the tibial trial insert system 1 is well-known to those skilled in the art.

With respect to said height and/or spacing adjustment, each shim 500, 600, 700 of said plurality of shims is configured to be slidable between the connector element 301 and the base element 302, in particular between an inferior surface 307 of the connector element 301 and a superior surface 308 of the base element 302.

For that purpose, a single shim or a plurality of stacked shims can be inserted between the connector element 301 and the base element 302. In other words, a single shim or more than a single shim of the plurality of shims can be used in combination to provide for an additional spacing between the bearing component 100 and the plate component 200.

For adjusting the proximal/distal spacing, the surgeon arranges the spacing adjustment assembly 300 between the bearing component 100 and the plate component 200 and engages the connector element 301 with the bearing component and the base element 302 with the plate component, respectively, in a manner described below in more detail. The actual spacing adjustment is achieved by inserting at least one of the shims 500, 600, 700 between the connector element 301 and the base element 302.

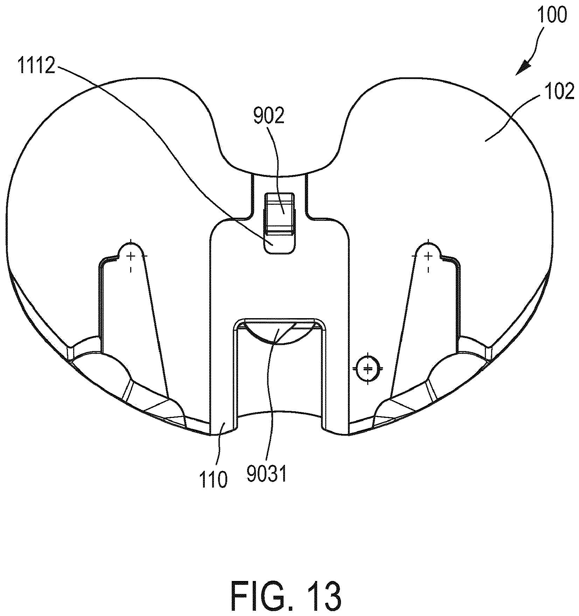

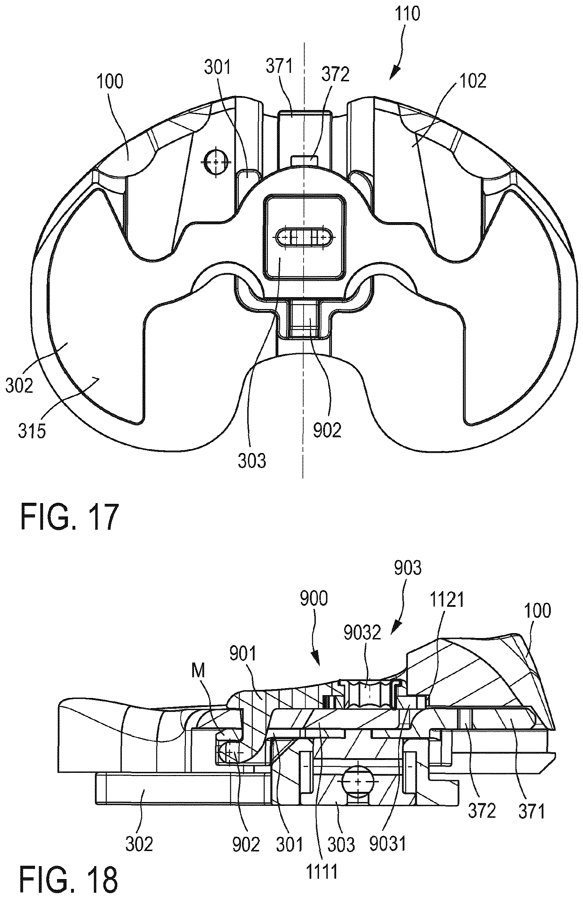

For removable engagement with the connector element 301, the bearing component 100 comprises an engagement portion 110 (see in particular figs. 13, 17). The engagement portion 110 is countersunk into the inferior surface 102 of the bearing component 100 in the proximal direction. In the illustrated embodiment, the engagement portion 110 and the connector element are configured to form a plug-in connection. The connector element 301 and the engagement portion 110 can be plugged together in anterior/posterior direction. In the engaged state, relative movement between the connector element 301 and the bearing component 100 is inhibited at least in proximal/distal direction and in medial/lateral direction.

For improved engagement between the connector element 301 and the engagement portion 110, the connector element 301 comprises a spring portion 380. The spring portion 380 serves as a fixture/stop for the bearing component 100 and prevents the bearing component 100 from unintended disengagement from the connector element 301. The spring portion 380 is configured to generate an audible effect upon reaching a fully engaged state with the engagement portion 110. Said audible effect indicates a correct position engagement of the bearing component on the spacing adjustment assembly 300.

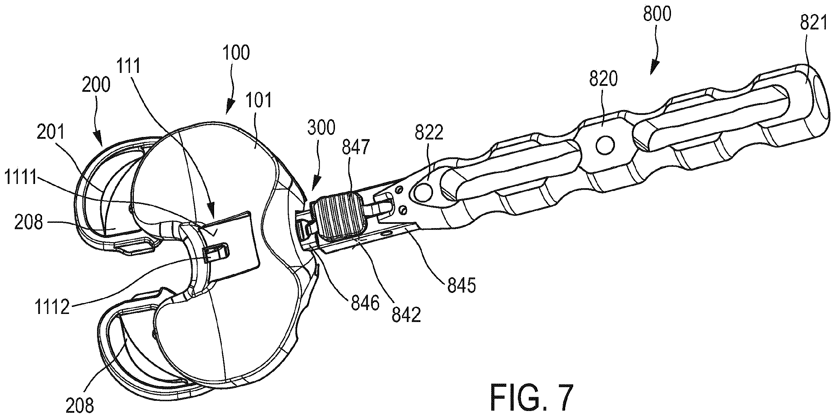

The base element 302 has a peripheral wall 324 extending between an inferior surface 315 and the superior surface 308. Moreover, the base element 302 has an anterior side 325, a posterior side 326, a lateral side 327 and a medial side 328. The peripheral wall 324 defines an outer contour (without reference sign). For removable engagement with the base element 302, the plate component 200 has a receiving recess 208 (see in particular fig. 1, 7) defining an inner contour (without reference sign) which is at least in portions complementary to the outer contour of the base element 302. In an engaged state, i.e. when the base element 302 is received in the receiving recess 208, the base element 302 is restrained in anterior/posterior direction and lateral/medial direction.

The coupling arrangement C forms a telescopic mechanism which is extendable in proximal direction and retractable in distal direction. The telescopic mechanism is configured to movably and captively couple the connector element 301 with the base element 302.

The telescopic mechanism comprises a cylinder element 303 which is fixedly connected to the connector element 301 and slidably received in a receiving bore 310 of the base element 302. The cylinder element 303 and the receiving bore 310 form a single-stage telescopic cylinder of the telescopic mechanism.

The cylinder element 303 and the receiving bore 310 extend coaxially in the proximal/distal direction.

The cylinder element 303 has a distal radial collar 313. The bore 310 of the base element 302 has a proximal radial collar 314. The distal radial collar 313 of the cylinder element 303 and the proximal radial collar 314 of the base element 302 form an end-stop for limiting the proximal movability of the cylinder element 303 within the bore 310.

The cylinder element 303 and the bore 310 each comprise a non-circular cross-section. In the illustrated embodiment, the cross-section of the cylinder element and the cross-section of the receiving bore 310 are each rectangular. The non-circular cross-sections prevent a relative rotation between the cylinder element 303 and the bore 310 and thus between the connector element 301 and the base element 302.

The cylinder element 303 is fixedly connected to the connector element 301. The connector element 301 comprises a pin hole 322. The cylinder element 303 comprises a pin portion 321. The pin portion 321 protrudes from a proximal end of the cylinder element 303 in proximal direction and is fixedly inserted into the pin hole 322. The pin portion 321 and the pin hole 322 can form an press fit and/or can be bonded together by means of a bonding connection.

The telescopic mechanism C comprises a latching device L configured for releasably latching the cylinder element 303 in a fully retracted position relative to the base element 302. Releasably latching the telescopic mechanism C in the fully retracted position, i.e. with minimal proximal/distal height, stabilizes and compacts the spacing adjustment assembly 300 which allows for an easy and safe insertion between the bearing component 100 and the plate component 200 during surgery.

In the illustrated embodiment, the latching device L comprises at least one resilient latching member 390 connected to the cylinder element 303 and at least one latching portion 394 formed on the base element 302. The resilient latching member 390 and the latching portion 394 are configured for a latching interaction to releasably lock the cylinder element 303 to the base element 302.

The latching connection between the resilient latching member 390 and the latching portion 394 is releasable by pulling the connector element 301 in proximal direction.

In the illustrated embodiment, the cylinder element 303 comprises at least one receiving bore 396 and the resilient latching member 390 comprises at least one head portion 391 and at least one compression spring member 392. The head portion 391 and the compression spring member 392 are both received in said receiving bore 396. In the illustrated embodiment, the receiving bore 396 extends in medial/lateral direction.

The head portion 391 extends between a first end 3911 and a second end 3912, wherein the first end 3911 is configured to latch with the latching portion 394, and wherein the compression spring member 392 acts on the second end 3912. The compression spring member 392 is configured to bias the head portion 391 into a latching position in which the first end 3911 protrudes from the receiving bore 396 to latch with the latching portion 394.

In the illustrated embodiment, the resilient latching member 390 comprises said (first) head portion 391 and a second head portion 393 opposite in medial/lateral direction. Moreover, the base element 302 comprises said (first) latching portion 394 and a second latching portion 395 opposite in medial/lateral direction. The compression spring member 392 is located between the first head portion 391 and the second head portion 393 and acts on their respective second ends 3912, 3932. The second head portion 393 is received in the receiving bore 396. The compression spring member 396 biases the first head portion 391 into the described latching position in which the first end 3911 of the first head portion 391 protrudes from the receiving bore to latch with the first latching portion 394. The compression spring member 392 also biases the second head portion 393 into a latching position in which a first end 3931 of the second head portion 393 protrudes from the receiving bore 396 to latch with the second latching portion 395.

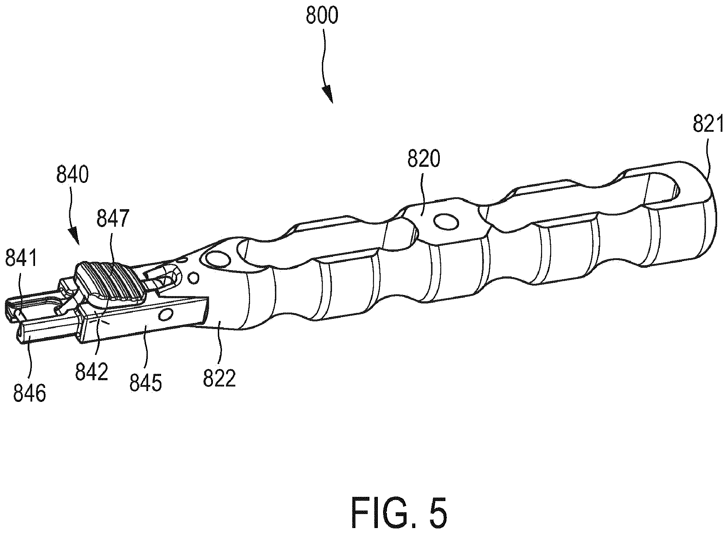

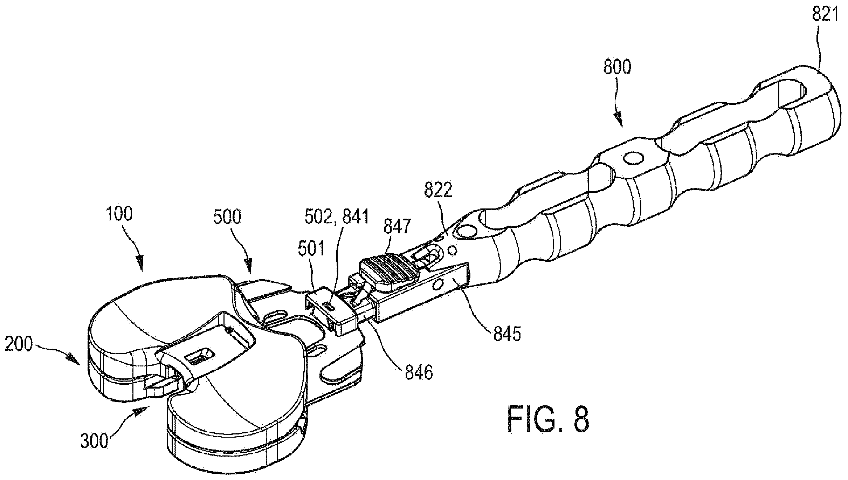

In the illustrated embodiment, the tibial trial insert system 1 further comprises a handling instrument 800 as shown in figs. 5 to 8. The handling instrument 800 comprises an elongated shaft 820 and a coupling mechanism 840.

The elongated shaft 820 extends between a proximal end 821 and a distal end 822.

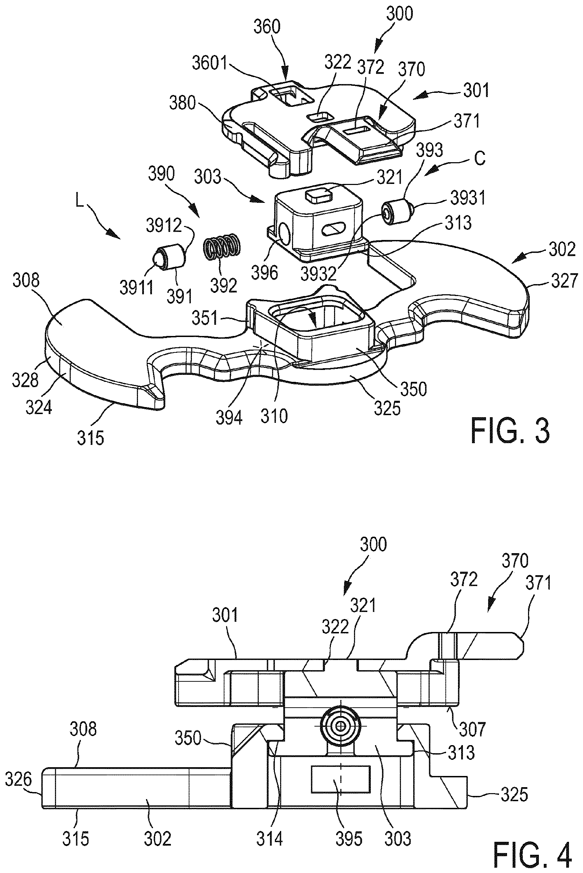

The coupling mechanism 840 is disposed at the distal end 822 and configured to releasably couple to an anterior coupling portion 370 (see figs. 3, 4) of the spacing adjustment assembly 300.

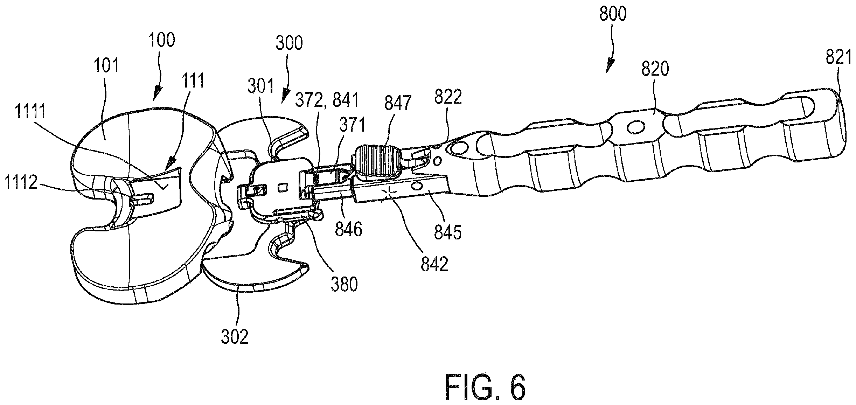

When releasably coupled to the anterior coupling portion 370, the handling instrument 800 allows for a simple and ergonomic engagement of the spacing adjustment assembly 300 with the bearing component 100 (see fig. 6). After engaging the spacing adjustment assembly 300 with the bearing component 100 the handling instrument, with the coupling mechanism 840 still coupled to the anterior coupling portion 370, can be used to insert the assembly 100, 300 into the patient's knee and to engage the base element 302 of the spacing adjustment assembly 300 with the plate component 200 (see fig. 7).

In the illustrated embodiment, the coupling mechanism 840 comprises a housing 845, a coupling finger 841, a coupling protrusion 846, a spring element 842 arranged inside the housing 845 and therefore not visible in the drawings, and a release button 847.

The release button 847 is biased by means of the spring element 842 and configured for manual operation, i.e. the release button 847 can be pressed against the bias of the spring element 842 by using a thumb. A distal end of the release button 847 forms the coupling finger 841. The coupling finger 841 is movable in proximal/distal direction by means of a movement of the release button 847.

The coupling protrusion 846 protrudes distally from a distal end of the housing 845.

In the illustrated embodiment, the anterior coupling portion 370 of the spacing adjustment assembly 300 is formed on the connector element 301. The connector element 301 is made of a metal material. The connection between the coupling mechanism 840 and the anterior coupling portion has therefore an improved strength and stability in comparison to a coupling of the coupling mechanism to a coupling portion made of plastic material, e.g. the plastic made coupling portion formed on the bearing component.

In the illustrated embodiment, the anterior coupling portion 370 comprises a coupling protrusion 371 and a coupling opening 372. The coupling protrusion 371 is configured for positive engagement with the coupling protrusion 846 of the coupling mechanism 840. The coupling opening 372 is configured for positive engagement with the coupling finger 841 of the coupling mechanism 840.

In the illustrated embodiment, the coupling mechanism 840 is also configured for releasable coupling to an anterior coupling portion AP, AP' of each of the plurality of shims 500, 600, 700 (see figs. 9, 10, 11).

The shims 500, 600, 700 depicted in figs. 9, 10, 11 can be denoted as first shim 500, second shim 600 and third shim 700.

The anterior coupling portion AP' of the third shim 700 is basically identical to the anterior coupling portion 370 of the connector element 301 and has a coupling protrusion 701 and a coupling opening 702.

The coupling protrusions AP of the first shim 500 and the second shim 600 each have a coupling recess 501, 601 and a coupling opening 502, 602. The coupling recesses 501, 601 and the coupling openings 502, 602 are each identical in form and function. The coupling recesses 501, 601 are configured to receive the coupling protrusion 846 of the coupling mechanism 840. The coupling openings 502, 602 are configured to receive the coupling finger 841.

In the illustrated embodiment, each shim of the plurality of shims 500, 600, 700 comprises two spring elements S. Each spring element S comprises a latching protrusion P or latching interaction with a complementary latching portion 351 of the base element 302 (see fig. 3).

In the illustrated embodiment, the complementary latching portion 351 is disposed on or in an outer wall of a boss section 350 of the base element. Said boss section 350 comprises the receiving bore 310. In the drawings, only one complementary latching portion 351 is visible. However, the boss section 350 comprises two complementary latching portions 351 opposite in medial/lateral direction. The spring elements S with their coupling protrusions P prevent an unintended removal of the respective shim 500, 600, 700.

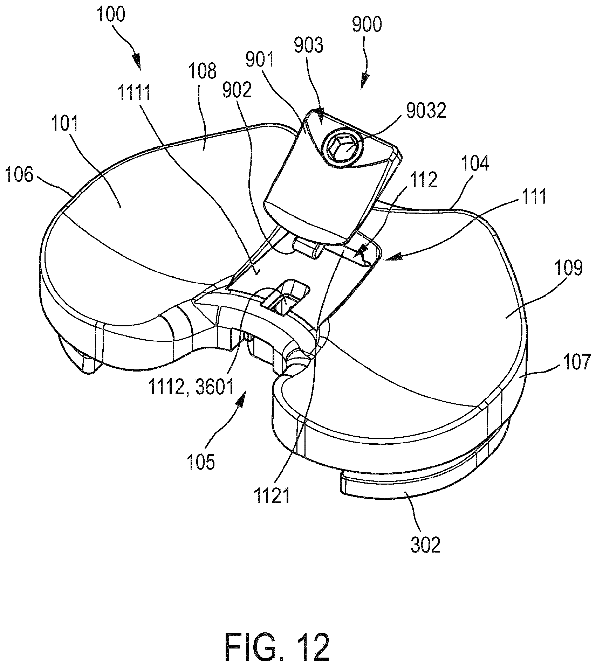

In the illustrated embodiment, the tibial trial insert system 1 further comprises a securing component 900 (see figs. 12 to 19). The securing component 900 is configured for securing the removable engagement between the bearing component 100 and the connector element 301 of the spacing adjustment assembly 300. For this purpose, the securing component 900 is configured to be supported on and/or in a support portion 111 of the bearing component 100 and configured for detachable attachment to an attachment portion 360 of the connector element 301.

In the illustrated embodiment, the support portion 111 of the bearing component 100 comprises a recessed support surface 1111 and a support opening 1112. The recessed support surface 1111 is sunk distally into the articulating surface 101. The support opening 1112 is located on the recessed support surface 1111 and extends between the support surface 1111 and the inferior surface 102 of the bearing component 100. The recessed support surface 1111 is located between the medial articulating surface portion 109 and the lateral articulating surface portion 108.

The attachment portion 360 of the connector element 301 comprises an attachment opening 3601. When the spacing adjustment assembly 300 is engaged with the bearing component 100, the attachment opening 3601 is positioned distally underneath and in line with the support opening 1112.

In the illustrated embodiment, the securing component 900 comprises a plug body 901 and a hook member 902.

The plug body 901 is configured to be plugged on the recessed support surface 1111. In the illustrated embodiment, an outer contour (without reference sign) of the plug body 901 is complementary to an inner contour (without reference sign) of the recessed support surface 1111.

The hook member 902 is configured to reach through the support opening 1112 and to positively engage with the attachment opening 3601 (see in particular figs. 13, 18).

In the illustrated embodiment, the securing component 900 further comprises a locking device 903 for releasably locking to a locking portion 112 of the bearing component 100. Releasably locking the securing component 900 to the bearing component 100 allows to easily attach and detach the securing component from the remaining components of the tibial trial insert system 1.

In the illustrated embodiment, the locking portion 112 of the bearing component 100 comprises a locking slit 1121 and the locking device 903 comprises a flat locking member 9031.

The locking slit 1121 extends in a transversal plane, i.e. in medial/lateral and in anterior/posterior direction and is located at an anterior end of the recessed support surface 1111.

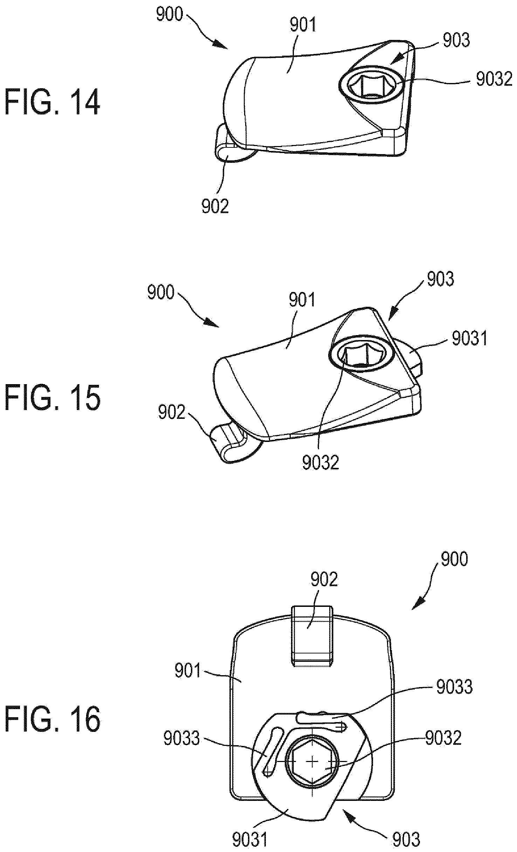

The flat locking member 9031 is configured to be rotatable relative to the plug body 901 and the hook member 902 between a locking position and a release position. When the plug body 901 is supported on the recessed support surface 111, the flat locking member is rotatable in said transversal plane of the locking slit 1121 between a locking position (see figs. 13, 15, 16, 18) and a release position (see fig. 14). In the locking position, the flat locking member 9031 positively engages the locking slit 1121. In the release position, the flat locking member 9031 does not positively engage the locking slit 1121.

In the illustrated embodiment, the flat locking member 9031 has a non-circular outer contour with two spring portions 9033. Said spring portions 9033 interact with an inner contour of a receiving recess (without reference sign) of the plug body 901 in which the flat locking member 9031 is received. The flat locking member 9031 is operatively connected with a screw member 9032. Turning the screw member 9032 rotates the flat locking member 9031 between the locking position and the release position.

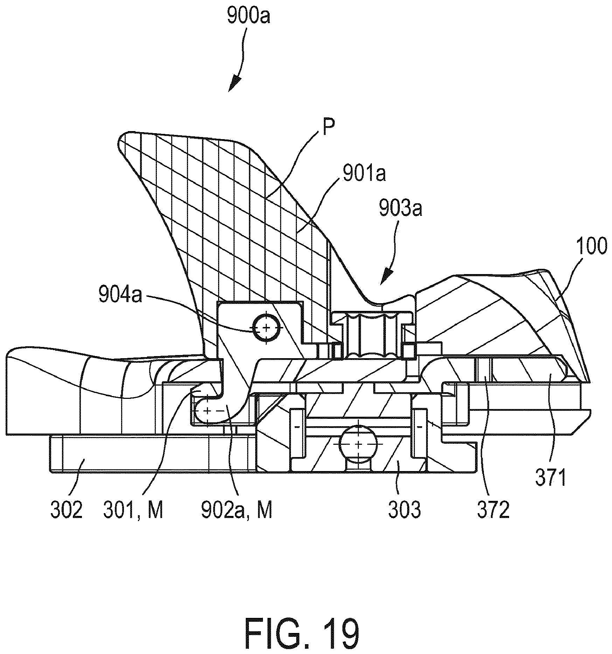

Fig. 19 illustrates the use of an alternative securing component 900a which can be considered as a variant of the securing component 900. The securing component 900a has a differently shaped plug body 901a. In particular, the plug body 901a forms a stem which is configured to interact with an intercondylar notch of a femoral component. The securing component 901a and the securing component 900 can be exchanged against one another. Choosing between the securing component 900 or the alternative securing component 900a allows the surgeon to change from one type of surgical procedure to another.

Referring to the securing component 900, the plug body 901 and the hook member 902 form a unitary one-piece design. In contrast, the plug body 901a and the hook member 902a of the securing component 900a are formed as separate parts. The plug body 901a is made of a plastic material. The hook member 902a is made of a metal material M. The hook member 902a is connected to the plug body 901a by means of a pin member 904a. The locking device 903a of the securing component 900a is identical with the locking device 903 of the securing component 900.

## Claims

1. A tibial trial insert system (1) comprising:
a bearing component (100) having a superior articulating surface (101) and an inferior surface (102);
a plate component (200) having a superior surface (201) and an inferior surface (202);
a spacing adjustment assembly (300) configured to be arrangeable between the bearing component (100) and the plate component (200), the spacing adjustment assembly (300) having at least one superior connector element (301) configured to removably engage with the bearing component (100), at least one inferior base element (302) configured to removably engage with the plate component (200), and having a coupling arrangement (C) movably coupling the connector element (301) and the base element (302) in proximal/distal direction and to a limited extent; and
a plurality of shims (500, 600, 700), each shim (500, 600, 700) configured to be slidable between the connector element (301) and the base element (302) to adjust a proximal/distal height of the spacing adjustment assembly (300), in order to thereby adjust a relative proximal/distal spacing between the bearing component (100) and the plate component (200).

2. The tibial trial insert system (1) according to claim 1, wherein the coupling arrangement (C) forms a telescopic mechanism which is extendable in proximal direction and retractable in distal direction and comprises at least one cylinder element (303) which is fixedly connected to the connector element (301) and slidably received in a receiving bore (310) of the base element (302).

3. The tibial trial insert system (1) according to claim 2, wherein the telescopic mechanism comprises a latching device (L) configured for releasably latching the cylinder element (303) in a fully retracted position relative to the base element (302).

4. The tibial trial insert system (1) according to claim 3, wherein the latching device (L) comprises at least one resilient latching member (390) connected to the cylinder element (303) and at least one latching portion (394) formed on the base element (302) and configured for latching interaction with the resilient latching member (390) to releasably latch the cylinder element (303) to the base element (302).

5. The tibial trial insert system (1) according to claim 4, wherein the cylinder element (303) comprises at least one receiving bore (396), wherein the resilient latching member (390) comprises at least one head portion (391) extending between a first end (3911) and a second end (3912), and at least one compression spring member (392), the head portion (391) and the compression spring member (392) both being received in said receiving bore (396), wherein the first end (3911) of the head portion (391) is configured to latch with the latching portion (394) of the base element (392), and wherein the compression spring member (392) acts on the second end (3912) of the head portion (391) to bias the head portion (391) into a latching position in which the first end (3911) of the head portion (391) protrudes from the receiving bore (396) of the cylinder element (303) to latch with the latching portion (394) of the base element (302).

6. The tibial trial insert system (1) according to claim 5, wherein the resilient latching member (390) comprises a first head portion (391) and a second head portion (393) opposite in medial/lateral direction, wherein the base element (302) comprises a first latching portion (394) and a second latching (395) portion opposite in medial/lateral direction, wherein the compression spring member (392) is located between said first head portion (391) and second head portion (393) and acts on their second ends (3912, 3932), thereby biasing the first head portion (391) into a latching position in which a first end (3911) of the first head portion (391) protrudes from the receiving bore (396) to latch with the first latching portion (394), and thereby biasing the second head portion (393) into a latching position in which a first end (3931) of the second head portion (393) protrudes from the receiving bore (396) to latch with the second latching portion (395).

7. The tibial trial insert system (1) according to any of the preceding claims, further comprising a handling instrument (800) having an elongated shaft (820) and a coupling mechanism (840) arranged distally on said elongated shaft (820), wherein the coupling mechanism (840) is configured for releasable coupling to an anterior coupling portion (370) of the spacing adjustment assembly (300).

8. The tibial trial insert system (1) according to claim 7, wherein the anterior coupling portion (370) is formed on the connector element (301) of the spacing adjustment assembly (300), and wherein the connector element (301) is made of a metal material.

9. The tibial trial insert system (1) according to claim 7 or 8, wherein the coupling mechanism (840) is configured for releasable coupling to an anterior coupling portion (AP, AP') of each of the plurality of shims (500, 600, 700).

10. The tibial trial insert system (1) according to any of the preceding claims, further comprising a securing component (900, 900a) configured to be supported on and/or in a support portion (111) of the bearing component (100) and configured for detachable attachment to an attachment portion (360) of the connector element (301) of the spacing adjustment assembly (300), thereby releasably securing the removable engagement between the bearing component (100) and the connector element (301).

11. The tibial trial insert system (1) according to claim 10, wherein the attachment portion (360) and/or the connector element (301) is made of a metal material.

12. The tibial trial insert system (1) according to claim 10 or 11, wherein the support portion (111) of the bearing component (100) comprises a recessed support surface (1111) which is sunk distally into the articulating surface (101) and a support opening (1112) extending between the support surface (1111) and an inferior surface (102) of the bearing component (100), wherein the attachment portion (360) of the connector element (301) comprises an attachment opening (3601) positioned distally underneath and in line with the support opening (1112), and wherein the securing component (900, 900a) comprises a plug body (901, 901a) configured to be plugged on the recessed support surface (1111) and a hook member (902, 902a) configured to reach through the support opening (1112) and to positively engage with the attachment opening (3061).

13. The tibial trial insert system (1) according to claim 12, wherein the hook member (902a) is made of a metal material and/or the plug body (901, 901a) is made of a plastic material.

14. The tibial trial insert system (1) according to any of claims 10 to 13, wherein the securing component (900, 900a) comprises a locking device (903, 903a) configured for releasably locking to a locking portion (112) of the bearing component (100).

15. The tibial trial insert system (1) according to claim 14, wherein the locking portion (112) of the bearing component (100) comprises a locking slit (1121) extending in a transversal plane and the locking device (903, 903a) of the securing component (900, 900a) comprises a flat locking member (9031) configured to be rotatable in said transversal plane relative to the securing component (900, 900a) between a locking position in which the flat locking member (9031) positively engages the locking slit (1121) and a release position in which the flat locking member (9031) does not positively engage the locking slit (1121).
